# EUROPEAN PATENT APPLICATION

(11) **EP 4 420 780 A1**
(43) Date of publication of application: **28.08.2024**
(21) Application number: 22882831.5
(22) Date of filing: 18.10.2022
(51) Int. Cl.: B01J 29/40, C07C 4/06, C07C 11/06, C07C 11/04

(54) **ZSM-5 MOLECULAR SIEVE CATALYST, PREPARATION METHOD THEREFOR AND APPLICATION THEREOF**

(30) Priority: 19.10.2021 CN 202111217885
(71) Applicant: China Petroleum & Chemical Corporation, Beijing 100728 (CN); Shanghai Research Institute of Petrochemical Technology, SINOPEC, Shanghai 201208 (CN)
(72) Inventor: TENG, Jiawei, Shanghai 201208 (CN); REN, Liping, Shanghai 201208 (CN); ZHAO, Guoliang, Shanghai 201208 (CN); SHI, Jing, Shanghai 201208 (CN); XIE, Zaiku, Shanghai 201208 (CN)
(74) Representative: karo IP
(86) International application number: PCT/CN2022/125833
(87) International publication number: WO 2023/066225

(57) **Abstract**

The present invention relates to a ZSM-5 molecular sieve catalyst, a preparation method therefor and an application thereof. In the catalyst, the ratio of the amount of skeleton aluminum located at the intersection of the straight pore channels and the sinusoidal pore channels to the amount of skeleton aluminum inside the straight pore channels and the sinusoidal pore channels is 1.4:1-10:1, the silica-alumina molar ratio SiO₂/Al₂O₃ is 80-1500, and the microporous pore volume may account for 70% to 92% of the total pore volume. When used for producing propylene and ethylene by olefin catalytic cracking, the catalyst of the present invention has characteristics of a low reaction hydrogen transfer index, a high stability, a high conversion rate of raw material olefin, and a high selectivity of products of propylene and ethylene.

## Description

### Technical field

The present invention relates to a field of catalytic cracking, specifically relates to a ZSM-5 molecular sieve catalyst and a preparation method therefor, as well as an application in increasing a propylene and ethylene production by an olefin catalytic cracking.

### Background technology

Propylene and ethylene are important basic raw materials in the petrochemical industry. Driven by a rapid growth in the demand for polyolefins and their derivatives, the demand for propylene and ethylene has continued to be strong and growing rapidly in recent years. Therefore, they are considered as products with a great market potential. Mixed C4 and more olefins are by-products of ethylene plants and FCC units in refineries, and can usually only be used as low value-added products such as a liquefied gas fuel. Further processing them into propylene and ethylene and fully utilizing this considerable amount of valuable olefin resources, undoubtedly have a significant effect on promoting the economic and technological development. Raw materials containing olefins can be converted into ethylene and propylene by olefin catalytic cracking technologies. With the continuous innovation and development of development technologies, the olefin catalytic cracking technology has achieved significant results in increasing the production of propylene and ethylene, greatly improving the production efficiency, as has a profound impact on the development of the petrochemical industry, and helps to promote the subsequent innovation and development of the petrochemical production technology. Catalyst, as a core technology in olefin catalytic cracking reactions, has been extensively studied by many scholars using various preparation methods. Researches focus on preparing catalysts with high activity and selectivity, improving the molecular sieve selectivity by regulating acid properties, optimizing and controlling the pore structure of molecular sieves. Moreover, the reduction of the generation of by-products and carbon deposits, the modification of element, the loading amount and the modification method would all directly affect the catalytic performance and the product distribution.

A catalyst used for the olefin cracking, with molecular sieves such as hydrogen type ZSM-5, ZSM-11 or SAPO-34 as active components and an inert gas as a heat carrier and a diluent, greatly benefits the improvement of various indicators in the reaction. However, the presence of water in the reaction is detrimental to a long-term use of the catalyst. Under a high-temperature hydrothermal condition, an acidic molecular sieve catalyst would usually undergo a severe skeleton dealumination, resulting in a rapid decrease in the catalyst acid density and an irreversible loss of the catalyst activity. At the same time, due to the strong acidity of molecular sieves, during the production of propylene and ethylene by the olefin cracking, side reactions such as an olefin oligomerization chain growth, a hydrogen transfer and an aromatization may occur, and even coking may occur in the pores of molecular sieve catalysts, covering reaction active centers and resulting in a rapid deactivation of the catalysts. EP0109059A1 discloses a method for cracking C₄-C₁₂ olefins to produce propylene, wherein the ZSM-5 or ZSM-11 molecular sieve is used as the catalyst. US6307117 discloses a method for cracking C₄-C₁₂ olefins to produce propylene and ethylene, wherein the active component of the catalyst as used is a ZSM-5 molecular sieve without protonic acid and with IB group metals. The olefin cracking catalysts reported in the above documents all have varying degrees of defects such as the poor product selectivity, the poor catalyst stability, the easy coking and deactivation and the inability to meet a long-term operation, thus making them difficult to achieve industrialization.

### Contents of the invention

The technical problem to be solved by the present invention is to provide a ZSM-5 molecular sieve catalyst and a preparation method therefor as well as an application of the catalyst in increasing a propylene and ethylene production by olefin catalytic cracking, in response to the problems of the poor catalyst stability and the low selectivity of propylene and ethylene in the production of propylene and ethylene by olefin catalytic cracking in the existing technologies. The catalyst of the present invention, when used for producing propylene and ethylene by olefin catalytic cracking, has characteristics of a low reaction hydrogen transfer index, a high stability, a high conversion rate of raw material of olefins, and a high selectivity of products of propylene and ethylene.

The first aspect of the present invention provides a ZSM-5 molecular sieve catalyst, wherein a ratio of an amount of skeleton aluminum located at an intersection of a straight pore channel and a sinusoidal pore channel to an amount of skeleton aluminum inside the straight pore channel and the sinusoidal pore channel is 1.4:1 to 10:1, and a silica-alumina molar ratio SiO₂/Al₂O₃ of the ZSM-5 molecular sieve is 80 to 1500.

In the above technical solution, in the catalyst, the ratio of the amount of skeleton aluminum located at the intersection of the straight pore channel and the sinusoidal pore channel to the amount of skeleton aluminum inside the straight pore channel and the sinusoidal pore channel is preferably 1.4:1 to 4:1, and more preferably 1.5:1 to 3:1.

In the above technical solution, within the ratio range of the amount of skeleton aluminum located at the intersection of the straight pore channel and the sinusoidal pore channel to the amount of skeleton aluminum inside the straight pore channel and the sinusoidal pore channel in the catalyst, the non-limiting specific point values can be 1.4:1, 1.5:1, 1.6:1, 1.7:1, 1.8:1, 1.9:1, 2.0:1, 2.1:1, 2.2:1, 2.3:1, 2.4:1, 2.5:1, 2.6:1, 2.7:1, 2.8:1, 2.9:1, 3.0:1, 3.2:1, 3.5:1, 4.0:1, and the like.

In the above technical solution, the silica-alumina molar ratio SiO₂/Al₂O₃ of the ZSM-5 molecular sieve in the catalyst is preferably 80 to 1000, more preferably 96 to 1000, even more preferably 150 to 1000, further more preferably 200 to 1000, and even further more preferably 250 to 1000.

In the above technical solution, a microporous pore volume preferably accounts for 70% to 92%, more preferably 75% to 90%, and even more preferably 80% to 90% of a total pore volume in the catalyst.

In the above technical solution, the non-limiting specific point values of the proportion of the microporous pore volume to the total pore volume can be 70%, 72%, 73%, 74%, 76%, 78%, 80%, 82%, 84%, 86%, 88%, 90%, 92%, and the like.

In the above technical solution, the total pore volume of the catalyst is preferably 0.01-1.2mL/g, more preferably 0.1-0.8mL/g.

In the above technical solution, the ZSM-5 molecular sieve catalyst preferably includes following components in parts by weight:
a) 90 to 100 parts, preferably 92 to 99 parts of a hydrogen type ZSM-5 molecular sieve;
b) 0-5 parts, preferably 0.5-3.0 parts of a rare earth element; and
c) 0-5 parts, preferably 0.5-5.0 part of an alkaline earth metal element.

In the above technical solution, the silica-alumina molar ratio SiO₂/Al₂O₃ of the hydrogen type ZSM-5 molecular sieve is preferably 80-1500, more preferably 80-1000, even more preferably 96-1000, still even more preferably 150-1000, further more preferably 200-1000, even further more preferably 250-1000.

In the above technical solution, the rare earth element is preferably at least one selected from La, Ce, Pr and Nd.

In the above technical solution, the alkaline earth metal element is preferably at least one selected from Mg, Ca, Sr and Ba.

In the above technical solution, the weight content of a binder, based on the weight of the catalyst, in the ZSM-5 molecular sieve catalyst is preferably 5% or less, more preferably 2% or less, and further preferably 0.5% or less. Furthermore, the ZSM-5 molecular sieve catalyst is preferably a binder-free ZSM-5 molecular sieve catalyst.

In the above technical solution, the ZSM-5 molecular sieve catalyst is preferably an olefin catalytic cracking catalyst.

The second aspect of the present invention provides a method for preparing the above ZSM-5 molecular sieve catalyst, comprising following steps:
(1) preparing a ZSM-5 molecular sieve raw powder;
(2) mixing and kneading the molecular sieve raw powder obtained in step (1) with a binder for molding to form a catalyst precursor after drying;
(3) subjecting the catalyst precursor obtained in step (2) to a third hydrothermal crystallization in the presence of a third template agent, an ammonium exchange to obtain the ZSM-5 molecular sieve catalyst.

In the above technical solution, optionally, step (4): loading a rare earth metal and/or an alkaline earth metal onto the ZSM-5 molecular sieve obtained in step (3) to obtain a metal-containing ZSM-5 molecular sieve catalyst, is included.

In the above technical solution, the method for preparing the ZSM-5 molecular sieve raw powder in step (1) preferably includes:
(1.1) mixing a first template agent, a first aluminum source, a silicon source, a first alkali source with water for a first hydrothermal crystallization;
(1.2) mixing a second aluminum source, a second template agent, a second alkali source with a mixture obtained after crystallization in step (1.1) to obtain the ZSM-5 molecular sieve raw powder after a second hydrothermal crystallization.

In the above technical solution, the first template agent in step (1.1) is preferably at least one of tetrapropylammonium bromide, tetrapropylammonium hydroxide, tetraethyl ammonium chloride and aqueous ammonia; the first aluminum source is preferably at least one of aluminum nitrate, aluminum sulfate and aluminum phosphate; the silicon source is preferably at least one of water glass, tetraethyl orthosilicate and silica sol; the first alkali source is preferably at least one of sodium hydroxide and potassium hydroxide. The first hydrothermal crystallization can be carried out under the self-generated pressure in a stainless steel autoclave. The water is preferably deionized water. The conditions for the first hydrothermal crystallization are preferably as follows: a crystallization temperature of 80-150 °C, a crystallization time of 2-10h. In step (1.1), the molar ratio of the first template agent based on NH₄⁺, the first aluminum source based on Al₂O₃, the silicon source based on SiO₂, the first alkali source based on OH⁻, and water based on H₂O was: NH₄⁺: Al₂O₃: SiO₂: OH⁻: H₂O=0.2 ~ 0.3: 0.0005 ~ 0.008: 1: 0.2 ~ 0.4: 15 ~ 20, preferably 0.2 ~ 0.3: 0.0005 ~ 0.001: 1: 0.2 ~ 0.4: 15 ~ 20.

In the above technical solution, the second aluminum source in step (1.2) is preferably at least one of potassium aluminum sulfate and sodium meta-aluminate. The aluminum potassium sulfate can be a hydrate, such as aluminum potassium sulfate dodecahydrate. The second template agent is preferably at least one of n-butylamine, hexane diamine and pyridine. The second alkali source is preferably at least one of sodium hydroxide and potassium hydroxide. The molar ratio of the silicon source added in step (1.1) to the total amount of the second aluminum source added in step (1.2) and the first aluminum source added in step (1.1), based on SiO₂/Al₂O₃, is preferably 80-1500, more preferably 80-1000. The amount of the second aluminum source based on Al₂O₃ added in step (1.2) preferably accounts for 30% or more, more preferably 40% or more, of the total weight of the second aluminum source based on Al₂O₃ in step (1.2) and the first aluminum source based on Al₂O₃ in step (1.1).

In the above technical solution, the molar ratio of the second template agent to the second aluminum source, based on NH₄⁺/Al₂O₃, in step (1.2) is preferably 200-500:1. The second alkaline source is used for controlling the pH value of the system to be preferably 8 to 10. The conditions for the second hydrothermal crystallization are preferably as follows: a crystallization temperature of 120-200 °C, a crystallization time of 10-100h. The second hydrothermal crystallization can be carried out under the self-generated pressure in a stainless steel autoclave.

In the above technical solution, the product obtained after the second hydrothermal crystallization can be washed, dried, calcined to obtain a ZSM-5 molecular sieve raw powder. The washing can be carried out with deionized water. The drying conditions are preferably as follows: a drying temperature of 80-100 °C , drying time of 10-20h. The calcination conditions are preferably as follows: a calcination temperature of 500-650 °C, and calcination time of 8-15h.

In step (2) of the above technical solution, the binder is preferably a silicon compound or a silicon compound and an aluminum compound. The aluminum compound is preferably at least one selected from aluminum oxide and aluminum sol, and the silicon compound is preferably at least one selected from white carbon black and silicon sol. In the binder, the silicon compound is calculated based on silicon dioxide and the aluminum compound is calculated based on aluminum oxide. The amount of the silicon compound may account for 10% to 100% by weight or 50% to 100% by weight. The added amount of the binder, based on the sum of the weights of aluminum oxide and silicon dioxide, is preferably 8% to 45%, more preferably 10% to 40%, of the total weight of the molecular sieve raw powder and the binder. The molding in step (2) can be carried out with conventional molding methods, such as an extrusion molding and the like. In step (2), an appropriate amount of water can be added according to the molding requirement. The drying conditions are preferably as follows: a drying temperature of 80-120 °C, drying time of 5-10h.

In the above technical solution, the third template agent in step (3) is preferably at least one of aqueous ammonia, ethylamine, ethylenediamine, triethylamine, n-butylamine, hexane diamine, tetrapropylammonium bromide and tetrapropylammonium hydroxide. The third hydrothermal crystallization is preferably an crystallization carried out by placing the catalyst precursor obtained in step (2) in a steam containing the third template agent. The weight ratio of the third template agent to the catalyst precursor is preferably 1 to 3:1, and the crystallization is preferably carried out at 130 to 200 °C for 20 to 100h. The third hydrothermal crystallization is preferably carried out with a gas-solid crystal transformation method, that is, the catalyst precursor is placed on an intermediate partition net of a crystallization kettle, and an aqueous solution containing the third template agent is placed below the intermediate partition net, and under a crystallization condition, a steam is formed from the aqueous solution containing the third template agent below to carry out a crystal transformation treatment on the catalyst precursor. The third hydrothermal crystallization can be carried out under the self-generated pressure in a stainless steel autoclave. The weight ratio of the third template agent to water in the aqueous solution of the third template agent is preferably 1 to 2:1.

In the above technical solution, the product obtained from the third hydrothermal crystallization can be washed, dried, calcined. The drying conditions are preferably as follows: a drying temperature of 80-100 °C, and drying time of 10-20h. The calcination conditions are preferably as follows: a calcination temperature of 450-600 °C, and calcination time of 5-10h.

In the above technical solution, the ammonium exchange in step (3) is preferably placing the product obtained from the crystallization of the catalyst precursor in an ammonium salt aqueous solution for an ammonium exchange, washing, drying. The ammonium salt is preferably one or more selected from the group consisting of ammonium chloride, ammonium nitrate and ammonium sulfate. The weight content of the ammonium salt in the ammonium salt aqueous solution is preferably 5% to 10%. The temperature for the ammonium exchange is preferably 80-90 °C ; the ammonium exchange frequency can be 3-6 times. The product obtained after the ammonium exchange is calcined; the calcination conditions are preferably as follows: a calcination temperature of 500-600 °C , and calcination time of 4-8 h.

In the above technical solution, step (4) is an optional step determined based on the constitution of the catalyst. The loading method of the rare earth metal and/or the alkaline earth metal may be an impregnation method. Preferably, the catalyst contains a rare earth metal and an alkaline earth metal, and the loading process is as follows: first, a ZSM-5 molecular sieve loaded with a rare earth metal is obtained, and then a ZSM-5 molecular sieve loaded with a rare earth metal and an alkaline earth metal is obtained, namely a metal-containing ZSM-5 molecular sieve catalyst. The impregnation method is preferably an isovolumetric impregnation method. A molecular sieve is subjected to an isovolumetric impregnation in a solution containing a rare earth metal salt or a solution containing an alkaline earth metal salt for 3-10h, dried at 60-100°C for 10-20h, calcined at 450-600°Cfor 8-10h. The weight concentration of the rare earth metal in the rare earth metal salt solution is 0.2% to 5%. The rare earth element is preferably at least one selected from La, Ce, Pr and Nd; the weight concentration of the alkaline earth metal in the alkaline earth metal salt solution is preferably 0.2% to 5%. The alkaline earth metal element is preferably at least one selected from Mg, Ca, Sr and Ba.

The third aspect of the present invention provides an application of the above ZSM-5 molecular sieve catalyst in the production of propylene and ethylene by olefin catalytic cracking, or a method for producing propylene and ethylene by using the above ZSM-5 molecular sieve catalyst in the olefin catalytic cracking.

In the above technical solution, the process for production of propylene and ethylene by olefin catalytic cracking can be as follows: contacting an olefin raw material with the above ZSM-5 molecular sieve catalyst for reaction to obtain propylene and ethylene products.

In the above technical solution, at least one of C4 to C6 olefins is preferably used as raw material, and the reaction conditions are preferably as follows: a reaction temperature of 400-600 °C, more preferably 420-580 °C, a reaction pressure of 0-0.3 MPa, more preferably 0.01-0.2 MPa, a weight space velocity of 1-50 h⁻¹, more preferably 2-40 h⁻¹.

In the above technical solution, propylene and ethylene are produced from the raw material through a catalyst bed layer, and the reaction hydrogen transfer index is preferably less than 9.6%, more preferably less than 7%; the reaction hydrogen transfer index herein is the yield weight ratio of propane and propylene in the product.

In the prior art, the process of increasing the production of propylene and ethylene by olefin catalytic cracking involves problems of a low selectivity of propylene and ethylene and a poor catalyst stability. This is mainly because the olefin cracking reaction network is complex, includes a single-molecule cracking, a double-molecule cracking and a trimolecular cracking. The process involves multiple steps such as an olefin isomerization, an oligomerization, a cracking, a dehydroaromatization, a hydrogen transfer reaction, an alkylation, an coking and the like, wherein the degree of the hydrogen transfer reaction is a key factor affecting the rest side reactions and the selectivity of products. Ordinary low silica-alumina ratio ZSM-5 molecular sieves have more acid centers, which, after being used in reaction, leads to an increase in side reactions, a low product selectivity, a blockage of the pores of the molecular sieve with by-products, thereby resulting in a decrease in the catalyst activity and ultimately the catalyst deactivation. However, high silica-alumina ratio ZSM-5 molecular sieves have fewer active centers and an uneven activity distribution, resulting in a lower reaction activity and a poor catalyst stability. The inventor found through research that the ZSM-5 molecular sieve catalyst, when used for producing propylene and ethylene by olefin catalytic cracking, exhibits a good activity and a good selectivity, and also a further enhanced stability, when the amount of the skeleton aluminum at the intersection of the straight pore channels and the sinusoidal pore channels is significantly larger than the amount of the skeleton aluminum inside the straight pore channels and the sinusoidal pore channels and the ZSM-5 molecular sieve catalyst has a relatively high silica-alumina ratio. In addition, an appropriate proportion of a microporous pore volume can help to improve the performance.

Compared with the prior art, the present invention has the following technical effects:
1. The ZSM-5 molecular sieve catalyst of the present invention has charactersitcs of a significantly larger amount of skeleton aluminum at the intersection of the straight pore channels and the sinusoidal pore channels compared to the amount of the skeleton aluminum inside the straight pore channels and the sinusoidal pore channels, as well as a relatively high silica-alumina ratio, as greatly improves the utilization rate of the catalyst active center, thereby making the products and the intermediates generated at the intersection of the pore channels more easily diffuse, dramatically reducing the occurrence of side reactions and the formation of carbon deposits, dramatically decreasing the reaction hydrogen transfer index, significantly improving the catalyst activity and the product selectivity and also further enhancing the stability.
2. During the preparation process of the ZSM-5 molecular sieve catalyst of the present invention, in particular during the synthesis process of the ZSM-5 molecular sieve raw powder, different aluminum sources and template agents are added in stages. The prepared molecular sieve catalyst has a composite pore channel structure, and after the crystal transformation treatment, the obtained catalyst basically does not contain any binder. When the amount of skeleton aluminum located at the intersection of the straight pore channels and the sinusoidal pore channels is significantly larger than the amount of skeleton aluminum inside the straight pore channels and the sinusoidal pore channels and the silica-alumina ratio is relatively high, the catalyst activity and the product selectivity are significantly improved, and the stability is also further increased. An appropriate proportion of the microporous pore volume further increases the performance.
3. The method for the production of ethylene and propylene by olefin catalytic cracking in the present invention effectively remedies the drawbacks of a high reaction hydrogen transfer index, a poor catalyst activity and a low selectivity of propylene and ethylene in the prior art. By using the catalyst of the present invention, the reaction hydrogen transfer index can be reduced to 9.6% or less, more preferably 7% or less, the conversion rate of the raw material of olefin is 71% or more, and the selectivity of the target products of propylene and ethylene is above 68%, preferably above 80%. After 75h of reaction, there is no significant change in the activity and the selectivity of the catalyst, indicating a good stability.

### Description of the drawings

Figure 1 is a skeleton aluminum ²⁷Al nuclear magnetic resonance spectrum of the catalyst obtained in Example 1. In Figure 1, line 1 represents the skeleton aluminum species at the intersection of the straight pore channels and the sinusoidal pore channels; line 2 represents the skeleton aluminum species inside the straight pore channels and the sinusoidal pore channels; line 3 represents the original curve before peak separation;
Figure 2 is a XRD spectrum of the catalyst obtained in Example 1;
Figure 3 is a skeleton aluminum ²⁷Al nuclear magnetic resonance spectrum of the the catalyst obtained in Comparative Example 5. In Figure 3, line 1 represents the skeleton aluminum species at the intersection of the straight pore channels and sinusoidal pore channels; line 2 represents the skeleton aluminum species inside the straight pore channels and the sinusoidal pore channels; line 3 represents the original curve before peak separation;
Figure 4 shows a XRD spectrum of the catalyst obtained in Comparative Example 5.

### Specific embodiments

The present invention is further illustrated through examples below.

In the present invention, the determination of the pore volume is carried out on a TriStar 3000 type physical adsorption instrument. After a vacuum treatment at 300 °C for 3h, a sample is placed in a testing instrument and liquid nitrogen is added for testing. The pore distribution of the sample is calculated using a Barret-Joyner-Helenda (BJH) model.

In the present invention, the instrument used for the ²⁷Al nuclear magnetic resonance characterization is Bruker's DSX 300 type nuclear magnetic resonance instrument. Refer to Al (H₂O)₆³⁺ in a saturated aluminum chloride solution for the chemical shift of ²⁷Al. The nuclear magnetic spectrum is obtained under the condition of the magic angle spinning speed of 4kHz. Therein, the skeleton aluminum located at the intersection of the straight pore channels and the sinusoidal pore channels in the catalyst corresponds to the peak of a chemical shift around 54 ppm (e.g. a range of 54 ± 0.2 ppm) in the ²⁷Al nuclear magnetic resonance spectrum, while the skeleton aluminum located inside the straight pore channels and the sinusoidal pore channels corresponds to the peak with a chemical shift around 56 ppm (e.g. a range of 56 ± 0.6 ppm) in the ²⁷Al nuclear magnetic resonance spectrum. The ratio of the amount of skeleton aluminum located at the intersection of the straight pore channels and the sinusoidal pore channels to the amount of skeleton aluminum located inside the straight pore channels and the sinusoidal pore channels is the ratio of the corresponding areas of the spectral peaks around 54ppm and 56ppm after peak separation according to the ²⁷Al nuclear magnetic resonance spectrum.

In the present invention, XRD analysis is performed on a Rigaku D/MAX-1400X type polycrystalline X-ray diffractometer using a graphite monochromator, Cu K α Ray, a tube voltage of 40 kV, a tube current of 40 mA, a scanning speed of 15°·min⁻¹, a scanning range 2 θ of 5-50 °.

In the present invention, the silica-alumina molar ratio SiO₂/Al₂O₃ is calculated by analyzing the elemental constitution of a solid sample using a Magix X type fluorescence spectrometer from Philips firm, Netherlands. The operating voltage is 40kV and the operating current is 40mA.

In the Examples and the Comparative Examples of the present invention, at least one of C4 to C6 olefins is used as a raw material for producing ethylene and propylene by catalytic cracking, wherein the hydrogen transfer index is a yield weight ratio of propane and propylene in the product; the conversion rate of raw material of olefin(%)=(1-the weight of olefin in the product/the weight of olefin in the raw material) ×100%; propylene and ethylene selectivity(%)=the sum of the weights of the produced propylene and ethylene in the product and/(the weight of olefin in the raw material-the weight of the remaining olefin after reaction)× 100%.

### [Example 1]

### (1) Preparation of a ZSM-5 molecular sieve raw powder

With tetrapropylammonium bromide as a first template agent, aluminum nitrate as a first aluminum source, silica sol as a silicon source, sodium hydroxide as a first alkali source, the molar ratio of tetrapropylammonium bromide based on NH₄⁺, aluminum nitrate based on Al₂O₃, silica sol based on SiO₂, sodium hydroxide based on OH⁻, and water based on H₂O was: NH₄⁺: Al₂O₃: SiO₂: OH⁻: H₂O=0.3:0.001:1:0.2:15. They were sufficiently mixed, stirred, and then transferred to an autoclave, crystallized at 100 °C for 8h, and then cooled for a later use. A second aluminum source of potassium aluminum sulfate dodecahydrate after excluding the first aluminum source was added according to a molar ratio of the silicon source to the total amount of the first aluminum source and the second aluminum source, based on SiO₂/Al₂O₃, of 300. A second template agent of n-butylamine was added according to a molar ratio of the second template agent to the second aluminum source, based on NH₄⁺/Al₂O₃, of 200:1, and was sufficiently mixed with the above crystallization solution; after adjusting the pH to 10 with a second alkali source of sodium hydroxide, the mixture was transferred to a stainless steel autoclave for a second hydrothermal crystallization at 120 °C for 100h. The synthesized product was washed with water, dried at 90 °C for 15h, and calcined at 600 °C for 10h to obtain the ZSM-5 molecular sieve raw powder.

### (2) Preparation of a catalyst precursor

100g of the above ZSM-5 molecular sieve raw powder, 25g of silica sol containing 40wt% SiO₂, and 0.056g of aluminium oxide were mixed, kneaded, extruded into strip for molding, and dried at 80 °C for 10h to obtain the catalyst precursor;

### (3) Preparation of a hydrogen type ZSM-5 molecular sieve

With ethylamine as a third template agent, a mixture of 30g of ethylamine and 30g of distilled water was added in advance in the reaction kettle, and 20g of the above prepared strip catalyst precursor was placed on a porous stainless steel net in the reaction kettle, and after being sealed, subjected to a gas-solid phase hydrothermal crystallization at 130 °C for 100h. After being withdrawn, the product was washed with distilled water, dried at 90 °C for 15h, and then calcined in an air atmosphere at 550 °C for 10h.

It was then subjected to an ammonium exchange three times in a 5wt% ammonium nitrate solution at 90 °C, and after drying, calcined in a muffle furnace at 500 °C for 4h to obtain the hydrogen type ZSM-5 molecular sieve.

### (4) Impregnation of metal components

The obtained hydrogen type ZSM-5 molecular sieve solid was placed in a 20g praseodymium nitrate solution with a Pr weight content of 1% for impregnation for 10h, dried at 100 °C for 10h, and calcined at 550 °C for 8h.

Finally, the above solid was impregnated in a 20g of magnesium nitrate solutiom with a Mg weight content of 2% for 8h, dried at 100 °C for 10h, and calcined at 550 °C for 8h to obtain a catalyst.

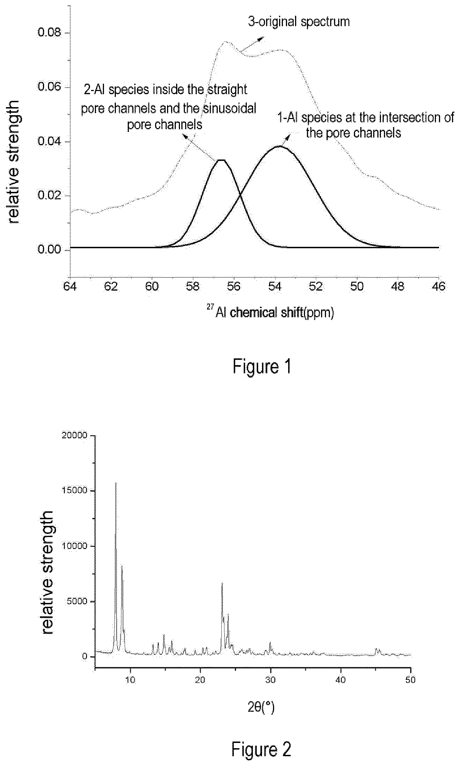

According to a physical adsorption test, the total pore volume of the catalyst was 0.3mL/g, with the microporous pore volume accounting for 86% of the total pore volume. According to aluminum nuclear magnetic resonance measurement, as shown in Figure 1, the ratio of the amount of skeleton aluminum located at the intersection of the straight pore channels and the sinusoidal pore channels to the amount of skeleton aluminum inside the straight pore channels and the sinusoidal pore channels was 2:1. The molar ratio SiO₂/Al₂O₃ of the catalyst was 282. Figure 2 is a XRD spectrum of the catalyst obtained in Example 1, indicating that the catalyst was a ZSM-5 molecular sieve catalyst with a binder content below 0.2%.

A fixed bed catalytic reaction device was used to evaluate the activity of the prepared catalyst for the reaction of producing propylene and ethylene by olefin catalytic cracking, with the raffinate mixed C4 (40% butane and 60% butene by weight) from an ethylene plant as a raw material. The process conditions used in the evaluation were as follows: loading 5g catalyst, a reaction temperature of 500 °C, a reaction pressure of 0.02 MPa, and a weight space velocity of 20 h⁻¹. The reaction results were: the C4 olefin conversion rate of 75%, the hydrogen transfer index of 6.5%, and the propylene and ethylene selectivity of 80.8%. The catalyst reacted for 80h without significant changes in activity and selectivity, demonstrating a good stability.

### Example 2

### (1) Preparation of a ZSM-5 molecular sieve raw powder

With tetrapropylammonium hydroxide as a first template agent, aluminum nitrate as a first aluminum source, tetraethyl orthosilicate as a silicon source, sodium hydroxide as a first alkali source, the molar ratio of tetrapropylammonium hydroxide based on NH₄⁺, aluminum nitrate based on Al₂O₃, tetraethyl orthosilicate based on SiO₂, sodium hydroxide based on OH⁻, and water based on H₂O was: NH₄⁺: Al₂O₃: SiO₂: OH⁻: H₂O=0.2:0.0005:1:0.4:20. They were sufficiently mixed, stirred, and then transferred to an autoclave, crystallized at 80 °C for 10h, then cooled for a later use. A second aluminum source of sodium aluminate after excluding the first aluminum source was added according to a molar ratio of the silicon source to the total amount of the first aluminum source and the second aluminum source, based on SiO₂/Al₂O₃, of 1000. A second template agent of pyridine was added according to a molar ratio of the second template agent to the second aluminum source, based on NH₄⁺/Al₂O₃, of 300:1, and was sufficiently mixed with the above crystallization solution; after adjusting pH to 8 with a second alkali source of sodium hydroxide, the mixture was transferred to a stainless steel autoclave for a second hydrothermal crystallization at 200°C for 10h. The synthesized product was washed with water, dried at 80°C for 20h, and calcined at 500 °C for 15h to obtain the ZSM-5 molecular sieve raw powder.

### (2) Preparation of a catalyst precursor

100g of the above ZSM-5 molecular sieve raw powder, 80g of white carbon black, and 0.68g of aluminum sol (containing 20% Al₂O₃ mass fraction) were mixed with 50g of water, kneaded, extruded into strip for molding, and dried at 120 °C for 5h to obtain the catalyst precursor;

### 3) Preparation of a hydrogen type ZSM-5 molecular sieve

With hexane diamine as a third template agent, a mixture of 60g of hexane diamine and 30g of distilled water was added in the reaction kettle in advance, and 20g of the above prepared strip catalyst precursor was placed on a porous stainless steel net in the reaction kettle, and after being sealed, crystallized at 150 °C for 80h. After being withdrawn, the product was washed with distilled water, dried at 100 °C for 10h, and calcined at 600 °C in an air atmosphere for 5h.

It was then subjected to an ammonium exchange three times in a 10% ammonium nitrate solution at 80°C, and after drying, calcined for 4 h in a muffle furnace at 550 °C to obtain the hydrogen type ZSM-5 molecular sieve.

### (4) Impregnation of metal components

The obtained hydrogen type ZSM-5 molecular sieve solid was placed in a 20g neodymium nitrate solution with a Nd weight content of 2% for 5h, dried at 60°C for 20h, and calcined at 600°C for 8h.

Finally, the above solid was impregnated in a 20g of calcium nitrate solution with a Ca weight content 0.8% for 10h, dried at 100 °C, and calcined at 500°C for 10h to obtain a catalyst. The XRD spectrum of the obtained catalyst was similar to Figure 2, indicating that it was a ZSM-5 molecular sieve catalyst with a binder content below 0.2%.

According to a physical adsorption test, the total pore volume of the catalyst was 0.4mL/g, with the microporous pore volume accounting for 90% of the total pore volume. According to aluminum nuclear magnetic resonance measurement, the ratio of the amount of skeleton aluminum located at the intersection of the straight pore channels and the sinusoidal pore channels to the amount of skeleton aluminum inside the straight pore channels and the sinusoidal pore channels was 1.5:1. The molar ratio SiO₂/Al₂O₃ of the catalyst was 925.

The catalyst evaluation method was the same as Example 1, and the reaction results were: the C4 olefin conversion rate of 73%, the hydrogen transfer index of 4.8%, and the propylene and ethylene selectivity of 85.6%. The catalyst reacted for 78h without significant changes in activity and selectivity, demonstrating a good stability.

### Example 3

### (1) Preparation of a ZSM-5 molecular sieve raw powder

With tetrapropylammonium bromide as a first template agent, aluminum nitrate as a first aluminum source, silica sol as a silicon source, sodium hydroxide as a first alkali source, the molar ratio of tetrapropylammonium bromide based on NH₄⁺, aluminum nitrate based on Al₂O₃, silica sol based on SiO₂, sodium hydroxide based on OH⁻, and water based on H₂O was: NH₄⁺: Al₂O₃: SiO₂: OH-: H₂O=0.3:0.001:1:0.2:15. They were sufficiently mixed and stirred, and then transferred to an autoclave, crystallized at 100 °C for 8h, and then cooled for a later use. A second aluminum source of potassium aluminum sulfate dodecahydrate after excluding the first aluminum source was added according to a molar ratio of the silicon source to the total amount of the first aluminum source and the second aluminum source, based on SiO₂/Al₂O₃, of 300. A second template agent of n-butylamine was added according to a molar ratio of the second template agent to the second aluminum source, based on NH₄⁺/Al₂O₃, of 200: 1, and was sufficiently mixed with the above crystallization solution; after adjusting the pH to 10 with a second alkali source of sodium hydroxide, the mixture was transferred to a stainless steel autoclave for a second hydrothermal crystallization at 120 °C for 100h. The synthesized product was washed with water, dried at 90 °C for 15h, and calcined at 600 °C for 10h to obtain the ZSM-5 molecular sieve raw powder.

### (2) Preparation of a catalyst precursor

100g of the above ZSM-5 molecular sieve raw powder, 25g of silica sol containing 40wt% SiO₂, and 0.056g of aluminium oxide were mixed, kneaded, extruded into strip for molding, and dried at 80 °C for 10h to obtain the catalyst precursor;

### (3) Preparation of a hydrogen type ZSM-5 molecular sieve

With ethylamine as a third template agent, a mixture of 30g of ethylamine and 30g of distilled water was added in advance in the reaction kettle, and 20g of the above prepared strip catalyst precursor was placed on a porous stainless steel net in the reaction kettle, and after being sealed, subjected to a gas-solid phase hydrothermal crystallization at 130 °C for 100h. After being withdrawn, the product was washed with distilled water, dried at 90 °C for 15h, and then calcined in an air atmosphere at 550 °C for 10h.

It was then subjected to an ammonium exchange three times in a 5wt% ammonium nitrate solution at 90 °C, and after drying, calcined in a muffle furnace at 500 °C for 4h to obtain the catalyst. The XRD spectrum of the obtained catalyst was similar to Figure 2, indicating that it was a ZSM-5 molecular sieve catalyst with a binder content below 0.2%.

According to a physical adsorption test, the total pore volume of the catalyst was 0.5mL/g, with the microporous pore volume accounting for 87% of the total pore volume. According to aluminum nuclear magnetic resonance measurement, the ratio of the amount of skeleton aluminum located at the intersection of the straight pore channels and the sinusoidal pore channels to the amount of skeleton aluminum inside the straight pore channels and the sinusoidal pore channels was 2:1. The molar ratio SiO₂/Al₂O₃ of the catalyst was 280.

The catalyst evaluation method was the same as Example 1, and the reaction results were: the C4 olefin conversion rate of 76%, the hydrogen transfer index of 9.6%, and the propylene and ethylene selectivity of 68.2%. The catalyst reacted for 82h without significant changes in activity and selectivity, demonstrating a good stability.

### Example 4

### (1) Preparation of a ZSM-5 molecular sieve raw powder

With tetrapropylammonium bromide as a first template agent, aluminum nitrate as a first aluminum source, silica sol as a silicon source, sodium hydroxide as a first alkali source, the molar ratio of tetrapropylammonium bromide based on as NH₄⁺, aluminum nitrate based on Al₂O₃, silica sol based on SiO₂, sodium hydroxide based on OH⁻, and water based on H₂O was: NH₄⁺: Al₂O₃: SiO₂: OH⁻: H₂O=0.3:0.001:1:0.2:15. They were sufficiently mixed and stirred, and then transferred to an autoclave, crystallized at 100 °C for 8h, and then cooled for a later use. A second aluminum source of potassium aluminum sulfate dodecahydrate after excluding the first aluminum source was added according to a molar ratio of the silicon source to the total amount of the first aluminum source and the second aluminum source, based on SiO₂/Al₂O₃, of 300. A second template agent of n-butylamine was added according to a molar ratio of the second template agent to the second aluminum source, based on NH₄⁺/Al₂O₃, of 200:1, and was sufficiently mixed with the above crystallization solution; after adjusting the pH to 10 with a second alkali source of sodium hydroxide, the mixture was transferred to a stainless steel autoclave for a second hydrothermal crystallization at 120 °C for 100h. The synthesized product was washed with water, dried at 90 °C for 15h, and calcined at 600 °C for 10 h to obtain the ZSM-5 molecular sieve powder.

### (2) Preparation of a catalyst precursor

100g of the above ZSM-5 molecular sieve raw powder, 25g of silica sol containing 40wt% SiO₂, and 0.056g of aluminium oxide were mixed, kneaded, extruded into strip for molding, and dried at 80 °C for 10h to obtain the catalyst precursor;

### (3) Preparation of a hydrogen type ZSM-5 molecular sieve

With ethylamine as a third template agent, a mixture of 30g of ethylamine and 30g of distilled water was added in advance in the reaction kettle, and 20g of the above prepared strip catalyst precursor was placed on a porous stainless steel net in the reaction kettle, and after being sealed, subjected to a gas-solid phase hydrothermal crystallization at 130 °C for 100h. After being withdrawn, the product was washed with distilled water, dried at 90 °C for 15h, and then calcined in an air atmosphere at 550 °C for 10h.

It was then subjected to an ammonium exchange three times in a 5wt% ammonium nitrate solution at 90 °C, and after drying, calcined in a muffle furnace at 500 °C for 4h to obtain the hydrogen type ZSM-5 molecular sieve.

### (4) Impregnation of metal components

The obtained hydrogen type ZSM-5 molecular sieve solid was placed in a 20g praseodymium nitrate solution with a Pr weight content of 1% for 10h, dried at 100 °C for 10h, and calcined at 550 °C for 8h to obtain a catalyst. The XRD spectrum of the obtained catalyst was similar to Figure 2, indicating that it was a ZSM-5 molecular sieve catalyst with a binder content below 0.2%.

According to a physical adsorption test, the total pore volume of the catalyst was 0.4mL/g, with the microporous pore volume accounting for 86% of the total pore volume. According to aluminum nuclear magnetic resonance measurement, the ratio of the amount of skeleton aluminum located at the intersection of the straight pore channels and the sinusoidal pore channels to the amount of skeleton aluminum inside the straight pore channels and the sinusoidal pore channels was 2:1. The molar ratio SiO₂/Al₂O₃ of the catalyst was 285.

The catalyst evaluation method was the same as Example 1, and the reaction results were: the C4 olefin conversion rate of 77.3%, the hydrogen transfer index of 8.8%, and the propylene and ethylene selectivity of 70.2%. The catalyst reacted for 76h without significant changes in activity and selectivity, demonstrating a good stability.

### Example 5

### (1) Preparation of a ZSM-5 molecular sieve raw powder

With tetrapropylammonium bromide as a first template agent, aluminum nitrate as a first aluminum source, silica sol as a silicon source, sodium hydroxide as a first alkali source, the molar ratio of tetrapropylammonium bromide based on NH₄⁺, aluminum nitrate based on Al₂O₃, silica sol based on SiO₂, sodium hydroxide based on OH⁻, and water based on H₂O was: NH₄⁺: Al₂O₃: SiO₂: OH⁻: H₂O=0.3:0.001:1:0.2:15. They were sufficiently mixed and stirred, and then transferred to an autoclave, crystallized at 100 °C for 8h, and then cooled for a later use. A second aluminum source of potassium aluminum sulfate dodecahydrate was added after excluding the first aluminum source according to a molar ratio of the silicon source to the total amount of the first aluminum source and the second aluminum source, based on SiO₂/Al₂O₃, of 300. A second template agent of n-butylamine was added according to the molar ratio of the second template agent to the second aluminum source, based on NH₄⁺/Al₂O₃, of 200:1, and was sufficiently mixed with the above crystallization solution; after adjusting the pH to 10 with a second alkali source of sodium hydroxide, the mixture was transferred to a stainless steel autoclave for a second hydrothermal crystallization at 120 °C for 100h. The synthesized product was washed with water, dried at 90 °C for 15 h, and calcined at 600 °C for 10 h to obtain the ZSM-5 molecular sieve powder.

### (2) Preparation of a catalyst precursor

100g of the above ZSM-5 molecular sieve raw powder, 25g of silica sol containing 40wt% SiO₂, and 0.056g of aluminium oxide were mixed, kneaded, extruded into strip for molding, and dried at 80 °C for 10h to obtain the catalyst precursor;

### (3) Preparation of a hydrogen type ZSM-5 molecular sieve

With ethylamine as a third template agent, a mixture of 30g of ethylamine and 30g of distilled water was added in advance in the autoclave, and 20g of the above prepared strip catalyst precursor was placed on a porous stainless steel net in the reaction kettle, and after being sealed, subjected to a gas-solid phase hydrothermal crystallization at 130 °C for 100h. After being withdrawn, the product was washed with distilled water, dried at 90 °C for 15h, and then calcined in an air atmosphere at 550 °C for 10h.

It was then subjected to an ammonium exchange three times in a 5wt% ammonium nitrate solution at 90 °C, and after drying, calcined in a muffle furnace at 500 °C for 4h to obtain the hydrogen type ZSM-5 molecular sieve.

### (4) Impregnation of metal components

The obtained hydrogen type ZSM-5 molecular sieve solid was placed in a 20 g magnesium nitrate solution with a Mg weight content of 2% for 8h, dried at 100 °C for 10h, and calcined at 550 °C for 8 h to obtain a catalyst. The XRD spectrum of the obtained catalyst was similar to Figure 2, indicating that it was a ZSM-5 molecular sieve catalyst with a binder content below 0.2%.

According to a physical adsorption test, the total pore volume of the catalyst was 0.6mL/g, with the microporous pore volume accounting for 83% of the total pore volume. According to aluminum nuclear magnetic resonance measurement, the ratio of the amount of skeleton aluminum located at the intersection of the straight pore channels and the sinusoidal pore channels to the amount of skeleton aluminum inside the straight pore channels and the sinusoidal pore channels was 1.8:1. The molar ratio SiO₂/Al₂O₃ of the catalyst was 282.

The catalyst evaluation method was the same as Example 1, and the reaction results were: the C4 olefin conversion rate of 75.8%, the hydrogen transfer index of 9.3%, and the propylene and ethylene selectivity of 69.5%. The catalyst reacted for 75h without significant changes in activity and selectivity, demonstrating a good stability.

### Example 6

### (1) Preparation of a ZSM-5 molecular sieve raw powder

With tetrapropylammonium bromide as a first template agent, aluminum sulfate as a first aluminum source, silica sol as a silicon source, sodium hydroxide as a first alkali source, the molar ratio of tetrapropylammonium bromide based on NH₄⁺, aluminum sulfate based on Al₂O₃, silica sol based on SiO₂, sodium hydroxide based on OH⁻, and water based on H₂O was: NH₄⁺: Al₂O₃: SiO₂: OH-: H₂O=0.2:0.005:1:0.4:20. They were sufficiently mixed and stirred, and then transferred to an autoclave, crystallized at 150 °C for 2h, and then cooled for a later use. A second aluminum source of aluminum potassium sulfate was added after excluding the first aluminum source according to a molar ratio of the silicon source to the total amount of the first aluminum source and the second aluminum source, based on SiO₂/Al₂O₃, of 100. A second template agent of n-butylamine was added according to a molar ratio of the second template agent to the second aluminum source, based on NH₄⁺/Al₂O₃, of 500:1, and was sufficiently mixed with the above crystallization solution; after adjusting the pH to 10 with a second alkali source of sodium hydroxide, the mixture was transferred to a stainless steel autoclave for a second hydrothermal crystallization at 150 °C for 40h. The synthesized product was washed with water, dried at 90 °C for 15h, and calcined at 600 °C for 10h to obtain the ZSM-5 molecular sieve powder.

### (2) Preparation of a catalyst precursor

115g of the above ZSM-5 molecular sieve raw powder, 10g of white carbon black, and 0.85g of aluminum sol (containing 20% Al₂O₃ mass fraction) were mixed with 40g of water, kneaded, extruded into strip for molding, and dried at 120 °C for 5h to obtain the catalyst precursor.

### (3) Preparation of a hydrogen type ZSM-5 molecular sieve

With hexane diamine as a third template agent, a mixture of 60g of hexane diamine and 30g of distilled water was added in the reaction kettle in advance, and 30g of the above prepared strip catalyst precursor was placed on a porous stainless steel net in the reaction kettle, and after being sealed, crystallized at 200 °C for 20h. After being withdrawn, the product was washed with distilled water, dried at 100 °C for 10h, and then calcined in an air atmosphere at 450 °C for 10h.

It was then subjected to an ammonium exchange 6 times in a 5% ammonium sulfate solution at 90 °C, and after drying, calcined in a muffle furnace at 600 °C for 4h to obtain the hydrogen type ZSM-5 molecular sieve.

### (4) Impregnation of metal components

The obtained hydrogen type ZSM-5 molecular sieve solid was impregnated in a 20g neodymium nitrate solution with a Ce weight content of 0.2% for 3h, dried at 100 °C for 10h, and calcined at 450 °C for 10h.

Finally, the above solid was impregnated in a 20g magnesium nitrate solution with a Mg weight content of 0.2% for 10h, dried at 100 °C, and calcined at 500 °C for 10h to obtain a catalyst. The XRD spectrum of the obtained catalyst was similar to Figure 2, indicating that it was a ZSM-5 molecular sieve catalyst with a binder content below 0.2%.

According to a physical adsorption test, the total pore volume of the catalyst was 0.8mL/g, with the microporous pore volume accounting for 90% of the total pore volume. According to aluminum nuclear magnetic resonance measurement, the ratio of the amount of skeleton aluminum located at the intersection of the straight pore channels and the sinusoidal pore channels to the amount of skeleton aluminum inside the straight pore channels and the sinusoidal pore channels was 3:1. The molar ratio SiO₂/Al₂O₃ of the catalyst was 98.

The catalyst evaluation method was the same as Example 1, and the reaction results were: the C4 olefin conversion rate of 76.3%, the hydrogen transfer index of 5.3%, and the propylene and ethylene selectivity of 84.5%. The catalyst reacted for 77h without significant changes in activity and selectivity, demonstrating a good stability.

### Example 7

### (1) Preparation of a ZSM-5 molecular sieve powder

With tetraethyl ammonium chloride as a first template agent, aluminum phosphate as a first aluminum source, water glass as a silicon source, sodium hydroxide as a first alkali source, the molar ratio of tetraethyl ammonium chloride based on NH₄⁺, aluminum phosphate based on Al₂O₃, water glass based on SiO₂, sodium hydroxide based on OH⁻, and water based on H₂O was: NH₄⁺: Al₂O₃: SiO₂: OH⁻: H₂O=0.2:0.001:1:0.4:20. They were sufficiently mixed and stirred, and then transferred to an autoclave, crystallized at 80 °C for 10h, and then cooled for a later use. A second aluminum source of sodium meta-aluminate was added after excluding the first aluminum source according to a molar ratio of the silicon source to the total amount of the first aluminum source and the second aluminum source, based on SiO₂/Al₂O₃, of 100. A second template agent of hexane diamine was added according to a molar ratio of the second template agent to the second aluminum source, based on NH₄⁺/Al₂O₃, of 200:1, and was sufficiently mixed with the above crystallization solution; after adjusting the pH to 10 with a second alkali source of sodium hydroxide, the mixture was transferred to a stainless steel autoclave for a second hydrothermal crystallization at 120 °C for 100h. The synthesized product was washed with water, dried at 100 °C for 10h, and calcined at 650 °C for 8h to obtain the ZSM-5 molecular sieve raw powder.

### (2) Preparation of a catalyst precursor

112g of the above ZSM-5 molecular sieve raw powder, 40g of white carbon black, and 3.4g of aluminum sol (containing 20% Al₂O₃ mass fraction) were mixed with 60g of water, kneaded, extruded into strip for molding, and dried at 80 °C for 10h to obtain the catalyst precursor;

### (3) Preparation of a hydrogen type ZSM-5 molecular sieve

With triethylamine as a third template agent, a mixture of 50g of triethylamine and 50g of distilled water was added in advance in the reaction kettle, 50g of the above prepared strip catalyst precursor was placed on a porous stainless steel net in the reaction kettle, and after being sealed, crystallized at 130 °C for 100h. After being withdrawn, the product was washed with distilled water, dried at 80 °C for 20h, and then calcined in an air atmosphere at 500 °C for 8h after drying out.

It was then subjected to an ammonium exchange 4 times in a 10% ammonium chloride solution at 80 °C, and after drying, calcined in a muffle furnace at 500 °C for 8h to obtain the hydrogen type ZSM-5 molecular sieve.

### (4) Impregnation of metal components

The obtained hydrogen type ZSM-5 molecular sieve solid was impregnated in a 20g lanthanum nitrate solution with a La weight content of 5% for 10h, dried at 100 °C for 12h, and calcined at 500 °C for 10h.

Finally, the above solid was impregnated in a 20g of barium nitrate solution with a Ba weight content of 0.5% for 10h, dried at 100 °C, and calcined at 500 °C for 10h to obtain a catalyst. The XRD spectrum of the obtained catalyst was similar to Figure 2, indicating that it was a ZSM-5 molecular sieve catalyst with a binder content below 0.2%.

According to a physical adsorption test, the total pore volume of the catalyst was 0.1mL/g, with the microporous pore volume accounting for 70% of the total pore volume. According to aluminum nuclear magnetic resonance measurement, the ratio of the amount of skeleton aluminum located at the intersection of the straight pore channels and the sinusoidal pore channels to the amount of skeleton aluminum inside the straight pore channels and the sinusoidal pore channels was 2:1. The SiO₂/Al₂O₃ molar ratio of the catalyst was 96. _{∘}

The catalyst evaluation method was the same as Example 1, and the reaction results were: the C4 olefin conversion rate of 71%, the hydrogen transfer index of 5.6%, and the propylene and ethylene selectivity of 80.3%. The catalyst reacted for 78h without significant changes in activity and selectivity, demonstrating a good stability.

### Example 8

### (1) Preparation of a ZSM-5 molecular sieve raw powder

With aqueous ammonia as a first template agent, aluminum nitrate as a first aluminum source, tetraethyl orthosilicate as a silicon source, sodium hydroxide as a first alkali source, the molar ratio of aqueous ammonia based on NH₄⁺, aluminum nitrate based on Al₂O₃, tetraethyl orthosilicate based on SiO₂, sodium hydroxide based on OH⁻, and water based on H₂O was: NH₄⁺: Al₂O₃: SiO₂: OH⁻: H₂O=0.3:0.008:1:0.2:20. They were sufficiently mixed, stirred, and then transferred to an autoclave, crystallized at 80 °C for 10h and then cooled for a later use. A second aluminum source of aluminum potassium sulfate was added after excluding the first aluminum source according to a molar ratio of the silicon source to the total amount of the first aluminum source and the second aluminum source, based on SiO₂/Al₂O₃, of 500. A second template agent of pyridine was added according to a molar ratio of the second template agent to the second aluminum source, based on NH₄⁺/Al₂O₃, of 400:1, and was sufficiently mixed with the above crystallization solution; after adjusting the pH to 8 with a second alkali source of sodium hydroxide, the mixture was transferred to a stainless steel autoclave for a second hydrothermal crystallization at 180 °C for 20h. The synthesized product was washed with water, dried at 90 °C for 15h, and calcined at 600 °C for 10h to obtain the ZSM-5 molecular sieve raw powder.

### (2) Preparation of a catalyst precursor

150g of the above ZSM-5 molecular sieve raw powder, 100g of white carbon black, and 1.7g of aluminum sol (containing 20% Al₂O₃ mass fraction) were mixed with 50g of water, kneaded, extruded into strip for molding, and dried at 120 °C for 5h to obtain the catalyst precursor;

### (3) Preparation of a hydrogen type ZSM-5 molecular sieve

With hexane diamine as a third template agent, a mixture of 50g of hexane diamine and 30g of distilled water was added in advance in the reaction kettle, and 20g of the above prepared strip catalyst precursor was placed on a porous stainless steel net in the reaction kettle, and after being sealed, crystallized at 150 °C for 80h. After being withdrawn, the product was washed with distilled water, dried at 100 °C for 10h, and then calcined in an air atmosphere at 600 °C for 5h.

It was subjected to an ammonium exchange three times in a 10% ammonium nitrate solution at 80 °C, and after drying, calcined in a muffle furnace at 550 °C for 4h to obtain the hydrogen type ZSM-5 molecular sieve.

### (4) Impregnation of metal components

The obtained hydrogen type ZSM-5 molecular sieve solid was placed in a 20g praseodymium nitrate solution with a Pr weight content of 1% for 5h, dried at 60 °C for 20h, and calcined at 600 °C for 8h.

Finally, the above solid was impregnated in a 20g of strontium nitrate solution with a Sr weight content of 0.3% for 10h, dried at 100 °C, and calcined at 500 °C for 10h to obtain a catalyst. The XRD spectrum of the obtained catalyst was similar to Figure 2, indicating that it was a ZSM-5 molecular sieve catalyst with a binder content below 0.2%.

According to a physical adsorption test, the total pore volume of the catalyst was 0.6mL/g, with the microporous pore volume accounting for 83% of the total pore volume. According to aluminum nuclear magnetic resonance measurement, the ratio of the amount of skeleton aluminum located at the intersection of the straight pore channels and the sinusoidal pore channels to the amount of skeleton aluminum inside the straight pore channels and the sinusoidal pore channels was 1.8:1. The molar ratio SiO₂/Al₂O₃ of the catalyst was 488.

The catalyst evaluation method was the same as Example 1, and the reaction results were: the C4 olefin conversion rate of 76.5%, the hydrogen transfer index of 5.1%, and the propylene and ethylene selectivity of 83.9%. The catalyst reacted for 80h without significant changes in activity and selectivity, demonstrating a good stability.

### Example 9

The reaction activity of the prepared catalyst for producing propylene and ethylene by olefin catalytic cracking was evaluated using the catalyst of Example 8, and using the nitrogen diluted pentene (the nitrogen to pentene volume ratio 1:1) as raw materials. The process conditions used in the evaluation were: loading 5g catalyst, a reaction temperature of 580 °C, a reaction pressure of 0.3 MPa, and a weight space velocity of 40h⁻¹. The reaction results were: the C5 olefin conversion rate of 80%, the hydrogen transfer index of 5.3%, the propylene and ethylene selectivity of 82.8%. The catalyst reacted for 78h without significant changes in activity and selectivity, demonstrating a good stability.

### Example 10

The reaction activity of the prepared catalyst for producing propylene and ethylene by olefin catalytic cracking was evaluated using the catalyst of Example 8, and using the nitrogen diluted hexene (the nitrogen to hexene volume ratio 1:1) as raw materials. The process conditions used in the evaluation were: loading 5g catalyst, a reaction temperature of 420 °C, a reaction pressure of 0.01 MPa, and a weight space velocity of 2h⁻¹. The reaction results were: the C6 olefin conversion rate of 72%, the hydrogen transfer index of 6.8%, and the propylene and ethylene selectivity of 80.3%. The catalyst reacted for 76h without significant changes in activity and selectivity, demonstrating a good stability.

### Comparative Example 1

### (1) Preparation of a ZSM-5 molecular sieve raw powder

With tetrapropylammonium bromide as a template agent, aluminum nitrate as an aluminum source, silica sol as a silicon source, sodium hydroxide as an alkali source, the molar ratio of tetrapropylammonium bromide based on NH₄⁺, aluminum nitrate based on Al₂O₃, silica sol based on SiO₂, sodium hydroxide based on OH⁻, and water based on H₂O was: NH₄⁺: Al₂O₃: SiO₂: OH⁻: H₂O = 0.3:0.001:1:0.2:15. They were sufficiently mixed, stirred and then transferred to an autoclave and crystallized at 100 °C for 8h. The synthesized product was washed with water, dried at 90 °C for 15h, and calcined at 600 °C for 10h to obtain the ZSM-5 molecular sieve powder.

### (2) Preparation of a catalyst precursor

100g of the above ZSM-5 molecular sieve raw powder, 25g of silica sol containing 40wt% SiO₂, and 0.056g of aluminium oxide were mixed, kneaded, extruded into strip for molding, and dried at 80 °C for 10h to obtain the catalyst precursor;

### (3) Preparation of a hydrogen type ZSM-5 molecular sieve

With ethylamine as a third template agent, a mixture of 30g of ethylamine and 30g of distilled water was added in advance in the reaction kettle, and 20g of the above prepared strip catalyst precursor was placed on a porous stainless steel net in the reaction kettle, and after being sealed, subjected to a gas-solid phase hydrothermal crystallization at 130 °C for 100h. After being withdrawn, the product was washed with distilled water, dried at 90 °C for 15h, and then calcined in an air atmosphere at 550 °C for 10h.

It was then subjected to an ammonium exchange three times in a 5wt% ammonium nitrate solution at 90 °C, and after drying, calcined in a muffle furnace at 500 °C for 4h to obtain the hydrogen type ZSM-5 molecular sieve.

### (4) Impregnation of metal components

The obtained hydrogen type ZSM-5 molecular sieve solid was impregnated in a 20g praseodymium nitrate solution with a Pr weight content of 1% for 10h, dried at 100 °C for 10h, and calcined at 550 °C for 8h.

Finally, the above solid was impregnated in a 20g magnesium nitrate solution with a Mg weight content of 2% for 8h, dried at 100 °C for 10h, and calcined at 550 °C for 8h to obtain a catalyst.

According to a physical adsorption test, the total pore volume of the catalyst was 0.6mL/g, with the microporous pore volume accounting for 68% of the total pore volume. According to aluminum nuclear magnetic resonance measurement, the ratio of the amount of skeleton aluminum located at the intersection of the straight pore channels and the sinusoidal pore channels to the amount of skeleton aluminum inside the straight pore channels and the sinusoidal pore channels was 1:1. The XRD spectrum was similar to Figure 2, and the binder content in the catalyst was less than 0.2%.

The catalyst evaluation method was the same as Example 1, and the reaction results were: the C4 olefin conversion rate of 76%, the hydrogen transfer index of 9.2%, the propylene and ethylene selectivity of 69.3%, and after 65h of the catalyst reaction, the catalyst activity and selectivity began to decrease.

### Comparative Example 2

### (1) Preparation of a ZSM-5 molecular sieve raw powder

With tetrapropylammonium bromide as a first template agent, aluminum nitrate as a first aluminum source, silica sol as a silicon source, sodium hydroxide as a first alkali source, the molar ratio of tetrapropylammonium bromide based on NH₄⁺, aluminum nitrate based on Al₂O₃, silica sol based on SiO₂, sodium hydroxide based on OH⁻, and water based on H₂O was: NH₄⁺: Al₂O₃: SiO₂: OH⁻: H₂O=0.3:0.001:1:0.2:15. They were sufficiently mixed and stirred, and then transferred to an autoclave, crystallized at 100 °C for 8h, and then cooled for a later use. A second aluminum source of aluminum nitrate was added after excluding the first aluminum source according to a molar ratio of the silicon source to the total amount of the first aluminum source and the second aluminum source, based on SiO₂/Al₂O₃, of 300. A second template agent of n-butylamine was added according to a molar ratio of the second template agent to the aluminum source, based on NH₄⁺/Al₂O₃, of 200:1, and was sufficiently mixed with the above crystallization solution; after adjusting the pH to 10 with a second alkali source of sodium hydroxide, the mixture was transferred to a stainless steel autoclave for a second hydrothermal crystallization at 120 °C for 100h. The synthesized product was washed with water, dried at 90 °C for 15h, and calcined at 600 °C for 10h to obtain the ZSM-5 molecular sieve raw powder.

### (2) Preparation of a catalyst precursor

50g of the above ZSM-5 molecular sieve raw powder, 25g of silica sol containing 40wt% SiO₂ and 0.056g of aluminium oxide were mixed, kneaded, extruded into strip for molding, and dried at 80 °C for 10h to obtain the catalyst precursor;

### (3) Preparation of a hydrogen type ZSM-5 molecular sieve

With ethylamine as a third template agent, a mixture of 30g of ethylamine and 30g of distilled water was added in advance in the reaction kettle, and 20g of the above prepared strip catalyst precursor was placed on a porous stainless steel net in the reaction kettle, and after being sealed, subjected to a gas-solid phase hydrothermal crystallization at 130 °C for 100h. After being withdrawn, the product was washed with distilled water, dried at 90 °C for 15h, and then calcined in an air atmosphere at 550 °C for 10h.

It was then subjected to an ammonium exchange three times in a 5wt% ammonium nitrate solution at 90 °C, and after drying, calcined in a muffle furnace at 500 °C for 4h to obtain the hydrogen type ZSM-5 molecular sieve.

### (4) Impregnation of metal components

The obtained hydrogen type ZSM-5 molecular sieve solid was impregnated in a 20g praseodymium nitrate solution with a Pr weight content of 1% for 10h, dried at 100 °C for 10h, and calcined at 550 °C for 8h.

Finally, the above solid was impregnated in a 20g of magnesium nitrate solution with a Mg weight content of 2% for 8h, dried at 100 °C for 10h, and calcined at 550 °C for 8h to obtain the catalyst.

According to a physical adsorption test, the total pore volume of the catalyst was 0.8mL/g, with the microporous pore volume accounting for 77% of the total pore volume. According to aluminum nuclear magnetic resonance measurement, the ratio of the amount of skeleton aluminum located at the intersection of the straight pore channels and the sinusoidal pore channels to the amount of skeleton aluminum inside the straight pore channels and the sinusoidal pore channels was 0.8:1. The SiO₂/Al₂O₃ molar ratio of the catalyst was 280. The XRD spectrum was similar to Figure 2, and the binder content in the catalyst was less than 0.2%.

The catalyst evaluation method was the same as Example 1, and the reaction results were: the C4 olefin conversion rate of 72%, the hydrogen transfer index of 7.3%, the propylene and ethylene selectivity of 75.5%, and after 70h of the catalyst reaction, the catalyst activity and selectivity began to decrease.

### Comparative Example 3

### (1) Preparation of a ZSM-5 molecular sieve raw powder

With tetrapropylammonium bromide as a first template agent, aluminum nitrate as a first aluminum source, silica sol as a silicon source, sodium hydroxide as a first alkali source, the molar ratio of tetrapropylammonium bromide based on NH₄⁺, aluminum nitrate based on Al₂O₃, silica sol based on SiO₂, sodium hydroxide based on OH⁻, and water based on H₂O was: NH₄⁺: Al₂O₃: SiO₂: OH⁻: H₂O=0.3:0.001:1:0.2:15. They were sufficiently mixed and stirred, and then transferred to an autoclave, crystallized at 100 °C for 8h, and then cooled for a later use. A second aluminum source of potassium aluminum sulfate dodecahydrate was added after excluding the first aluminum source according to a molar ratio of the silicon source to the total amount of the first aluminum source and the second aluminum source, based on SiO₂/Al₂O₃, of 300. A template agent of tetrapropylammonium bromide was added according to a molar ratio of the template agent to the second aluminum source, based on NH₄⁺/Al₂O₃, of 200:1, and was sufficiently mixed with the above crystallization solution; after adjusting the pH to 10 with a second alkali source of sodium hydroxide, the mixture was transferred to a stainless steel autoclave for a second hydrothermal crystallization at 120 °C for 100h. The synthesized product was washed with water, dried at 90 °C for 15h, and calcined at 600 °C for 10h to obtain the ZSM-5 molecular sieve raw powder.

### (2) Preparation of a catalyst precursor

100g of the above ZSM-5 molecular sieve raw powder, 25g of silica sol containing 40wt% SiO₂, and 0.056g of aluminium oxide were mixed, kneaded, extruded into strip for molding, and dried at 80 °C for 10h to obtain the catalyst precursor;

### (3) Preparation of a hydrogen type ZSM-5 molecular sieve

With ethylamine as a third template agent, a mixture of 30g of ethylamine and 30g of distilled water was added in advance in the reaction kettle, and 20g of the above prepared strip catalyst precursor was placed on a porous stainless steel net in the reaction kettle, and after being sealed, subjected to a gas-solid phase hydrothermal crystallization at 130 °C for 100h. After being withdrawn, the product was washed with distilled water, dried at 90 °C for 15h, and then calcined in an air atmosphere at 550 °C for 10h.

It was then subjected to an ammonium exchange three times in a 5wt% ammonium nitrate solution at 90 °C, and after drying, calcined in a muffle furnace at 500 °C for 4h to obtain the hydrogen type ZSM-5 molecular sieve.

### (4) Impregnation of metal components

The obtained hydrogen type ZSM-5 molecular sieve solid was impregnated in a 20g praseodymium nitrate solution with a Pr weight content of 1% for 10h, dried at 100 °C for 10h, and calcined at 550 °C for 8h.

Finally, the above solid was impregnated in a 20g magnesium nitrate solution with a Mg weight content of 2% for 8h, dried at 100 °C for 10h, and calcined at 550 °C for 8h to obtain a catalyst. The XRD spectrum of the obtained catalyst was similar to Figure 2, indicating that it was a ZSM-5 molecular sieve catalyst.

According to a physical adsorption test, the total pore volume of the catalyst was 0.28mL/g, with the microporous pore volume accounting for 73% of the total pore volume. According to aluminum nuclear magnetic resonance measurement, the ratio of the amount of skeleton aluminum located at the intersection of the straight pore channels and the sinusoidal pore channels to the amount of skeleton aluminum inside the straight pore channels and the sinusoidal pore channels was 1.2:1. The molar ratio SiO₂/Al₂O₃ of the catalyst was 285. The XRD was similar to Figure 2, and the binder content in the catalyst was less than 0.2%

The catalyst evaluation method was the same as Example 1, and the reaction results were: the C4 olefin conversion rate of 69.8%, the hydrogen transfer index of 7.2%, the propylene and ethylene selectivity of 71.8%, and after 72h of the catalyst reaction, the catalyst activity and selectivity began to decrease.

### Comparative Example 4

### (1) Preparation of a ZSM-5 molecular sieve raw powder

With tetrapropylammonium bromide as a first template agent, aluminum nitrate as a first aluminum source, silica sol as a silicon source, sodium hydroxide as a first alkali source, the molar ratio of tetrapropylammonium bromide based on NH₄⁺, aluminum nitrate based on Al₂O₃, silica sol based on SiO₂, sodium hydroxide based on OH⁻, and water based on H₂O was: NH₄⁺: Al₂O₃: SiO₂: OH⁻: H₂O=0.3:0.01:10:2:15. They were sufficiently mixed and stirred, and then transferred to an autoclave, crystallized at 100 °C for 8h, and then cooled for a later use. A second aluminum source of potassium aluminum sulfate dodecahydrate was added after excluding the first aluminum source according to a molar ratio of the silicon source to the total amount of the first aluminum source and the second aluminum source, based on SiO₂/Al₂O₃, of 50. A second template agent of n-butylamine was added according to a molar ratio of the second template agent and the second aluminum source, based on NH₄⁺/Al₂O₃, of 200:1, and was sufficiently mixed with the above crystallization solution; after adjusting the pH to 10 with a second alkali source of sodium hydroxide, the mixture was transferred to a stainless steel autoclave for a second hydrothermal crystallization at 120 °C for 100h. The synthesized product was washed with water, dried at 90 °C for 15h, and calcined at 600 °C for 10h to obtain the ZSM-5 molecular sieve raw powder.

### (2) Preparation of a catalyst precursor

100g of the above ZSM-5 molecular sieve raw powder, 25g of silica sol containing 40wt% SiO₂, and 0.056g of aluminium oxide were mixed, kneaded, extruded into strip for molding, and dried at 80 °C for 10h to obtain the catalyst precursor;

### (3) Preparation of a hydrogen type ZSM-5 molecular sieve

With ethylamine as a third template agent, a mixture of 30g of ethylamine and 30g of distilled water was added in advance in the reaction kettle, and 20g of the above prepared strip catalyst precursor was placed on a porous stainless steel net in the reaction kettle, and after being sealed, subjected to a gas-solid phase hydrothermal crystallization at 130 °C for 100h. After being withdrawn, the product was washed with distilled water, dried at 90 °C for 15h, and then calcined in an air atmosphere at 550 °C for 10h.

It was then subjected to an ammonium exchange three times in a 5wt% ammonium nitrate solution at 90 °C, and after drying, calcined in a muffle furnace at 500 °C for 4h to obtain the hydrogen type ZSM-5 molecular sieve.

### (4) Impregnation of metal components

The obtained hydrogen type ZSM-5 molecular sieve solid was impregnated in a 20g praseodymium nitrate solution with a Pr weight content of 1% for 10h, dried at 100 °C for 10h, and calcined at 550 °C for 8h.

Finally, the above solid was impregnated in a 20g magnesium nitrate solution with a Mg weight content of 2% for 8h, dried at 100 °C for 10h, and calcined at 550 °C for 8h to obtain the catalyst. The XRD spectrum of the obtained catalyst was similar to Figure 2, indicating that it was a ZSM-5 molecular sieve catalyst.

According to a physical adsorption test, the total pore volume of the catalyst was 0.3mL/g, with the microporous pore volume accounting for 80% of the total pore volume. According to aluminum nuclear magnetic resonance measurement, the ratio of the amount of skeleton aluminum located at the intersection of the straight pore channels and the sinusoidal pore channels to the amount of skeleton aluminum inside the straight pore channels and the sinusoidal pore channels was 1.8:1. The molar ratio of SiO₂/Al₂O₃ in the catalyst was 47. The XRD was similar to Figure 2, and the binder content in the catalyst was less than 0.2%.

The catalyst evaluation method was the same as Example 1, and the reaction results were: the C4 olefin conversion rate of 68.6%, the hydrogen transfer index of 12.3%, the propylene and ethylene selectivity of 62.5%. After 72h of the catalyst reaction, the catalyst activity and selectivity began to decrease.

### Comparative Example 5

### (1) Preparation of a ZSM-5 molecular sieve raw powder

With tetrapropylammonium bromide as a first template agent, aluminum nitrate as a first aluminum source, silica sol as a silicon source, sodium hydroxide as a first alkali source, the molar ratio of tetrapropylammonium bromide based on NH₄⁺, aluminum nitrate based on Al₂O₃, silica sol based on SiO₂, sodium hydroxide based on OH⁻, and water based on H₂O was: NH₄⁺: Al₂O₃: SiO₂: OH⁻: H₂O=0.3:0.001:1:0.2:15. They were sufficiently mixed and stirred, and then transferred to an autoclave, crystallized at 100 °C for 8h, and then cooled for a later use. A second aluminum source of potassium aluminum sulfate dodecahydrate was added after excluding the first aluminum source according to a molar ratio of the silicon source to the total amount of the first aluminum source and the second aluminum source, based on SiO₂/Al₂O₃, of 300. A second template agent of n-butylamine was added according to the molar ratio of the second template agent to the second aluminum source, based on NH₄⁺/Al₂O₃, of 200:1, and was sufficiently mixed with the above crystallization solution; after adjusting the pH to 10 with a second alkali source of sodium hydroxide, the mixture was transferred to a stainless steel autoclave for a second hydrothermal crystallization at 120 °C for 100h. The synthesized product was washed with water, dried at 90 °C for 15h, and calcined at 600 °C for 10h to obtain the ZSM-5 molecular sieve raw powder.

### (2) Preparation of a catalyst precursor

100g of the above ZSM-5 molecular sieve raw powder, 25g of silica sol containing 40wt% SiO₂, and 0.056g of aluminium oxide were mixed, kneaded, extruded into strip for molding, and dried at 80 °C for 10h to obtain the catalyst precursor.

### (3) Preparation of a hydrogen type ZSM-5 molecular sieve

20g of the above prepared strip catalyst precursor was calcined in an air atmosphere at 550 °C for 10h.

It was then subjected to an ammonium exchange three times in a 5wt% ammonium nitrate solution at 90 °C, and after drying, calcined in a muffle furnace at 500 °C for 4h to obtain the hydrogen type ZSM-5 molecular sieve.

### (4) Impregnation of metal components

The obtained hydrogen type ZSM-5 molecular sieve solid was impregnated in a 20g praseodymium nitrate solution with a Pr weight content of 1% for 10h, dried at 100 °C for 10h, and calcined at 550 °C for 8h.

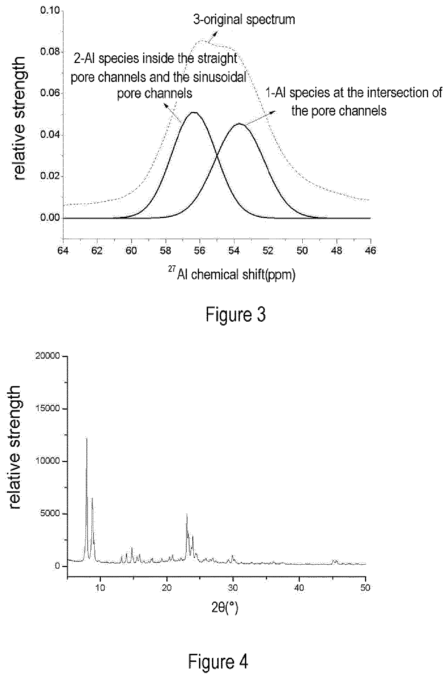

Finally, the above solid was impregnated in a 20g of magnesium nitrate solution with a Mg weight content 2% for 8h, dried at 100 °C for 10h, and calcined at 550 °C for 8h to obtain a catalyst. Figure 4 shows an XRD spectrum of the catalyst obtained in Comparative Example 5, indicating that the catalyst was a ZSM-5 molecular sieve catalyst with a binder content of 10%.

According to a physical adsorption test, the total pore volume of the catalyst was 0.1mL/g, with the microporous pore volume accounting for 68% of the total pore volume. According to aluminum nuclear magnetic resonance measurement, as shown in Figure 3, the ratio of the amount of skeleton aluminum located at the intersection of the straight pore channels and the sinusoidal pore channels to the amount of skeleton aluminum inside the straight pore channels and the sinusoidal pore channels was 0.8:1. The molar ratio SiO₂/Al₂O₃ of the catalyst was 282.

The catalyst evaluation method was the same as Example 1, and the reaction results were: the C4 conversion rate of 65%, the hydrogen transfer index of 15.8%, and the propylene and ethylene selectivity of 58.5%.

### Comparative Example 6

### (1) Preparation of a ZSM-5 molecular sieve raw powder

With tetrapropylammonium bromide as a template agent, aluminum nitrate as an aluminum source, silica sol as a silicon source, sodium hydroxide as an alkali source, the molar ratio of tetrapropylammonium bromide based on NH₄⁺, aluminum nitrate based on Al₂O₃, silica sol based on SiO₂, sodium hydroxide based on OH⁻, and water based on H₂O was: NH₄⁺: Al₂O₃: SiO₂: OH⁻: H₂O = 0.3:0.001:1:0.2:15. They were sufficiently mixed and stirred, and then transferred to an autoclave and crystallized at 100 °C for 8h. The synthesized product was washed with water, dried at 90 °C for 15h, and calcined at 600 °C for 10h to obtain the ZSM-5 molecular sieve raw powder.

### (2) Preparation of a catalyst precursor

100g of the above ZSM-5 molecular sieve raw powder, 25g silica sol containing 40wt% SiO₂, and 0.056g of aluminium oxide were mixed, kneaded, extruded into strip for molding, and dried at 80 °C for 10h to obtain the catalyst precursor.

### (3) Preparation of a hydrogen type ZSM-5 molecular sieve

20g of the above prepared strip catalyst precursor was calcined in an air atmosphere at 550 °C for 10h.

It was then subjected to an ammonium exchange three times in a 5wt% ammonium nitrate solution at 90 °C, and after drying, calcined in a muffle furnace at 500 °C for 4h to obtain the hydrogen type ZSM-5 molecular sieve.

### (4) Impregnation of metal components

The obtained hydrogen type ZSM-5 molecular sieve solid was impregnated in a 20g praseodymium nitrate solution with a Pr weight content of 1% for 10h, dried at 100 °C for 10h, and calcined at 550 °C for 8h.

Finally, the above solid was impregnated in a 20g magnesium nitrate solution with a Mg weight content of 2% for 8h, dried at 100 °C for 10h, and calcined at 550 °C for 8h to obtain a catalyst. The XRD spectrum of the obtained catalyst was similar to Figure 4, indicating that it was a ZSM-5 molecular sieve catalyst with a binder content of 10%.

According to a physical adsorption test, the total pore volume of the catalyst was 0.13mL/g, with the microporous pore volume accounting for 62% of the total pore volume. According to aluminum nuclear magnetic resonance measurement, the ratio of the amount of skeleton aluminum located at the intersection of the straight pore channels and the sinusoidal pore channels to the amount of skeleton aluminum inside the straight pore channels and the sinusoidal pore channels was 0.7:1. The SiO₂/Al₂O₃ molar ratio of the catalyst was 280.

The catalyst evaluation method was the same as Example 1, and the reaction results were: the C4 conversion rate of 62.5%, the hydrogen transfer index of 16.9%, and the propylene and ethylene selectivity of 53.5%.

As can be seen from Figures 1 and 3, the ZSM-5 molecular sieve catalyst of the present invention has a significantly higher ratio of the amount of skeleton aluminum located at the intersection of the straight pore channels and the sinusoidal pore channels to the amount of skeleton aluminum inside the straight pore channels and the sinusoidal pore channels than that of the conventional catalysts.

As can be seen from Figures 2 and 4, the crystallinity of the catalyst obtained in Example 1 of the present invention is significantly higher than that of the catalyst obtained in Comparative Example 5, and the ZSM-5 molecular sieve catalyst obtained in Comparative Example 5 contains a large amount of the binder component.

The specific embodiments of the present invention are described in detail as above, to which, however, the present invention is not limited. Within the scope of the technical concept of the present invention, simple variations can be made to the technical solution of the present invention, including combinations of various technical features in any other suitable ways. These simple variations and combinations should also be considered as the contents disclosed in the present invention, all of which fall within the scope of protection of the present invention.

## Claims

1. A ZSM-5 molecular sieve catalyst, wherein a ratio of an amount of skeleton aluminum located at an intersection of straight pore channels and sinusoidal pore channels to an amount of skeleton aluminum inside straight pore channels and sinusoidal pore channels is 1.4:1-10:1, preferably 1.4:1-4:1, more preferably 1.5:1-3:1, and a silica-alumina molar ratio SiO₂/Al₂O₃ of the ZSM-5 molecular sieve catalyst is 80-1500, preferably 80-1000, more preferably 96-1000, even more preferably 150-1000, and further more preferably 200-1000, even further more preferably 250-1000.

2. The catalyst according to claim 1, **characterized in that** a microporous pore volume of the catalyst accounts for 70% to 92% of a total pore volume, preferably 75% to 90%, and more preferably 80% to 90%.

3. The catalyst according to claim 1 or 2, **characterized in that** the total pore volume of the catalyst is 0.01-1.2 mL/g, preferably 0.1-0.8 mL/g.

4. The catalyst according to any one of claims 1-3, **characterized in** comprising following components in parts by weight:
a) 90 to 100 parts of a hydrogen type ZSM-5 molecular sieve, preferably 92 to 99 parts;
b) 0-5 parts of a rare earth element, preferably 0.5-3.0 parts; and
c) 0-5 parts of an alkaline earth metal element, preferably 0.5-5.0 parts.

5. The catalyst according to any one of claims 1-4, **characterized in that** a silica-alumina molar ratio SiO₂/Al₂O₃ of the hydrogen type ZSM-5 molecular sieve is 80-1500, preferably 80-1000, more preferably 96-1000, even more preferably 150-1000, further more preferably 200-1000, and even further more preferably 250-1000.

6. The catalyst according to any one of claims 4-5, **characterized in that** the rare earth element is at least one selected from La, Ce, Pr and Nd; and/or, the alkaline earth metal element is at least one selected from Mg, Ca, Sr and Ba.

7. The catalyst according to any one of claims 1-6, **characterized in that** in the ZSM-5 molecular sieve catalyst, based on the weight of the catalyst, a weight content of a binder is 5% or less, preferably 2% or less, further preferably 0.5% or less, and particularly preferably the ZSM-5 molecular sieve catalyst is binder-free.

8. A preparation method for the ZSM-5 molecular sieve catalyst according to any one of claims 1-7, comprising:
(1) preparing a ZSM-5 molecular sieve raw powder;
(2) mixing and kneading the molecular sieve raw powder obtained in step (1) with a binder for molding to form a catalyst precursor after drying;
(3) subjecting the catalyst precursor obtained in step (2) to a third hydrothermal crystallization in the presence of a third template agent, an ammonium exchange to obtain the ZSM-5 molecular sieve catalyst.

9. The preparation method according to claim 8, **characterized in that** the preparation method further comprises step (4): loading a rare earth metal and/or an alkaline earth metal onto the ZSM-5 molecular sieve obtained in step (3) to obtain a metal-containing ZSM-5 molecular sieve catalyst.

10. The preparation method according to claim 8 or 9, **characterized in that** the preparation method for the ZSM-5 molecular sieve raw powder in step (1) comprises:
(1.1) mixing a first template agent, a first aluminum source, a silicon source, a first alkali source with water for a first hydrothermal method;
(1.2) mixing a second aluminum source, a second template agent, a second alkali source with a mixture obtained after crystallization in step (1.1) to obtain the ZSM-5 molecular sieve raw powder after a second hydrothermal crystallization.

11. The preparation method according to claim 10, **characterized in that** the first template agent in step (1.1) is at least one of tetrapropylammonium bromide, tetrapropylammonium hydroxide, tetraethyl ammonium chloride and aqueous ammonia;
and/or, the first aluminum source in step (1.1) is at least one of aluminum nitrate, aluminum sulfate and aluminum phosphate;
and/or, conditions for the first hydrothermal crystallization in step (1.1) are as follows: a crystallization temperature of 80-150°C, a crystallization time of 2-10h;
and/or, in step (1.1), the molar ratio of the first template agent based on NH₄⁺, the first aluminum source based on Al₂O₃, the silicon source based on SiO₂, the first alkali source based on OH⁻, and water based on H₂O was: NH₄⁺: Al₂O₃: SiO₂: OH⁻: H₂O=0.2-0.3: 0.0005-0.008: 1: 0.2-4: 15-20, preferably 0.2-0.3: 0.0005-0.001: 1: 0.2-0.4: 15-20.

12. The preparation method according to claim 10 or 11, **characterized in that** the second aluminum source in step (1.2) is at least one of potassium aluminum sulfate and sodium meta-aluminate;
and/or, an amount of the second aluminum source, based on Al₂O₃, added in step (1.2) accounts for 30% or more, preferably 40% or more, of the total weight of the second aluminum source in step (1.2) and the first aluminum source in step (1.1), based on Al₂O₃;
and/or, the second template agent in step (1.2) is at least one of n-butylamine, hexane diamine and pyridine;
and/or, in step (1.2), a second alkali source is used to control pH value of the system to be from 8 to 10;
and/or, conditions for the second hydrothermal crystallization are as follows: a crystallization temperature of 120-200 °C, and a crystallization time of 10-100h.

13. The preparation method according to any one of claims 8-12, **characterized in that** the third template agent in step (3) is at least one of aqueous ammonia, ethylamine, ethylenediamine, triethylamine, n-butylamine, hexane diamine, tetrapropylammonium bromide and tetrapropylammonium hydroxide;
and/or, the third hydrothermal crystallization is a crystallization by placing the catalyst precursor obtained in step (2) in a steam containing the third template agent, a weight ratio of the third template agent to the catalyst precursor is 1-3:1, and the crystallization is carried out at 130 to 200°Cfor 20 to 200h.

14. Use of the catalyst as claimed in any one of claims 1-7 or prepared the by the preparation method according to any one of claims 8-13 in a production of propylene and ethylene by olefin catalytic cracking.

15. The use according to claim 14, **characterized in that** an olefin raw material is contacted with a ZSM-5 molecular sieve catalyst for reaction to obtain propylene and ethylene products, preferably at least one of C4 to C6 olefins is used as the raw material, reaction conditions are as follows: a reaction temperature of 400-600 °C , preferably 420-580 °C , a reaction pressure of 0-0.3 MPa, preferably 0.01-0.2 MPa, a weight space velocity of 1-50 h⁻¹, preferably 2-40 h⁻¹.
